# EUROPÄISCHE PATENTSCHRIFT

(11) **EP 1 216 982 B1**
(45) Veröffentlichungstag und Bekanntmachung des Hinweises auf die Patenterteilung: **05.03.2008**
(21) Anmeldenummer: 01127302.6
(22) Anmeldetag: 19.11.2001
(51) Int. Cl.: C07C 68/02, C07C 69/96, C08G 64/30

(54) **Verfahren zur Herstellung von Kohlensäurediarylestern**
Process for the preparation of diaryl esters of carbonic acid
Procédé de préparation d'esters diaryliques d'acide carbonique

(30) Priorität: 19.12.2000 DE 10063296
(43) Veröffentlichungstag der Anmeldung: 26.06.2002
(73) Patentinhaber: Bayer MaterialScience AG, 51368 Leverkusen (DE)
(72) Erfinder: Alewelt, Wolfgang, Dr., 47809 Krefeld (DE); Kühling, Steffen, Dr., 40670 Meerbusch (DE)

(56) Entgegenhaltungen:
- EP-A- 0 391 202
- EP-A- 0 423 008
- DE-A- 2 509 036
- DE-A- 2 804 227
- DE-A- 4 238 123
- GB-A- 1 483 889

## Beschreibung

Gegenstand der vorliegenden Erfindung ist ein Verfahren zur Herstellung von Kohlensäurediarylestern durch Reaktion von Monophenolen und Phosgen in einem inerten Lösungsmittel in Gegenwart von Alkali und einer Stickstoffbase in der Phasengrenzfläche bei niedrigen Reaktionstemperaturen.

Die Herstellung von Kohlensäurediarylestern durch den Phasengrenzflächenprozess ist prinzipiell in der Literatur bekannt, siehe z.B. Chemistry and Physics of Polycarbonates, Polymer Reviews, H. Schnell, Vol. 9, John Wiley and Sons, Inc. (1964), S. 50/51. Im US-P 4 016 190 wird ein Herstellungsverfahren von Kohlensäurediarylestern beschrieben, das bei Temperaturen > 65°C betrieben wird und bei dem Stickstoffbasen als Katalysatoren eingesetzt werden.

Als Nebenprodukte entstehen bei der Durchführung dieser Verfahren typischerweise Aryldialkylurethane entweder durch Reaktion von Phosgen mit den als Katalysatoren eingesetzten tertiären Aminen zum Carbaminsäurechlorid und anschließender Reaktion mit anwesendem Phenol zum Urethan (1.) oder durch Reaktion von Arylchlorkohlensäureester aus Phenol und Phosgen mit den tertiären Aminen (2.) gemäß folgendem, allgemeinen Reaktionsschema: 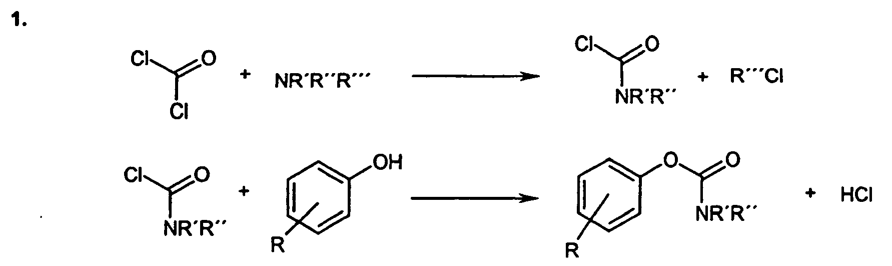 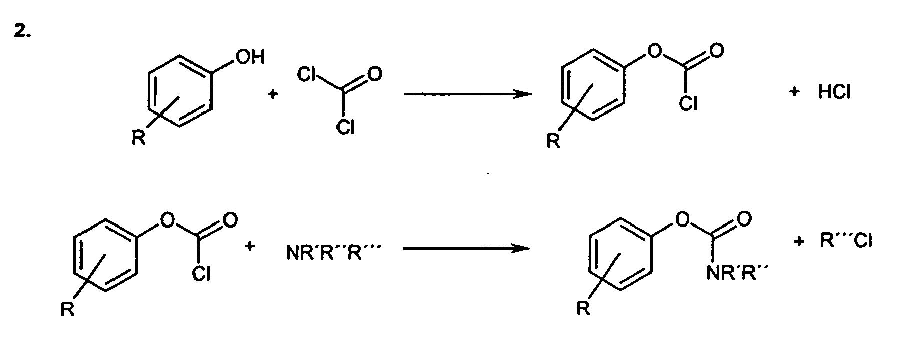 worin R die Reste gemäß Formel (I) (s.u.) bedeutet und R', R" und R"' für organische Reste allgemein stehen.

Diese Verbindungen stören beim Schmelzeumesterungsprozess zum Polycarbonat und reichem sich insbesondere in einem Kohlensäurediphenylesterkreislauf an. Daher ist die Unterdrückung der Bildung dieser Aryldialkylurethane bzw. deren Entfernung aus dem Kohlensäurediarylester besonders wichtig.

Die aus dem Stand der Technik bekannten Verfahren, ausgehend von Monophenolen und Phosgen in einem Lösungsmittel unter Zuhilfenahme einer Stickstoffbase als Katalysator sind hinsichtlich der Reinheit der erhaltenen Produkte, insbesondere bezüglich deren Chlor- und Urethangehalts jedoch nicht zufriedenstellend. Aus diesem Grund müssen die aus diesen Verfahren erhältlichen Produkte noch weiteren Reinigungsprozessen unterzogen werden.

Es stellte sich daher, ausgehend vom Stand der Technik, die Aufgabe, ein Verfahren bereitzustellen, welches chlor- und insbesondere nebenproduktarme, speziell urethanarme, Kohlensäurediarylester verfügbar macht.

Es wurde nun überraschenderweise gefunden, daß bei der Herstellung von Kohlensäurediarylestern durch Reaktion von Monophenolen und Phosgen in einem inerten Lösungsmittel im Phasengrenzflächenverfahren in Gegenwart von Alkali bei Anwendung einer Stickstoffbasenkatalyse bei niedrigen Reaktionstemperaturen gearbeitet werden muss, um eine hohe Produktreinheit des Kohlensäurediarylesters erhalten zu können und die genannten Nebenreaktionen zu vermeiden.

Die nach dem erfindungsgemäßen Verfahren einzusetzenden Katalysatoren sind tertiäre Amine, N-Alkylpiperidine oder Oniumsalze. Bevorzugt werden Tributylamin, Triethylamin und N-Ethylpiperidin verwendet. Ganz besonders bevorzugt wird N-Ethylpiperidin. Die Konzentrationen der Katalysatoren sind 0,0001 mol-% bis 0,1 mol-%, bevorzugt 0,01 mol-% bis 0,075 mol-%, bezogen auf das eingesetzte Phenol.

Das eingesetzte Alkali kann eine Alkalilauge (Na, K, Li, Ca-hydroxid) sein, bevorzugt Natronlauge, und wird im erfindungsgemäßen Verfahren vorzugsweise als 20 bis 55 gew.-%ige, besonders bevorzugt 30 bis 50 gew.-%ige Lösung eingesetzt.

Phosgen kann flüssig, gasförmig oder gelöst im inerten Lösungsmittel eingesetzt werden.

Die Monophenole zum Einsatz im erfindungsgemäßen Verfahren sind Phenole der Formel (I) 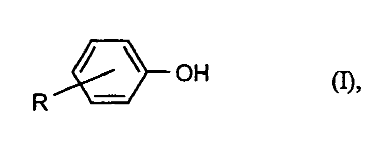 worin
- R: Wasserstoff, tert.-Butyl, oder ein verzweigter oder unverzweigter C₈- und/oder C₉-Alkylrest ist,
somit also Phenol selbst, p-tert.-Butylphenol, p-n-Octylphenol, p-iso-Octylphenol, p-n-Nonylphenol und p-iso-Nonylphenol. Bevorzugt ist Phenol.

Im erfindungsgemäßen Verfahren werden inerte organische Lösungsmittel verwendet, beispielsweise Dichlormethan, Toluol, die verschiedenen Dichlorethane und Chlorpropanverbindungen, Chlorbenzol und Chlortoluol, vorzugsweise wird Dichlormethan eingesetzt.

Die Reaktion kann sowohl diskontinuierlich (batchweise) als auch kontinuierlich, bevorzugt kontinuierlich, geführt werden und wird bevorzugt in einer Pfropfenströmung ohne große Rückvermischung durchgeführt. Dies kann beispielsweise in Rohrreaktoren geschehen. Die Durchmischung der zwei Phasen (wässrige und organische Phase) kann durch eingebaute Rohrblenden, statische Mischer und/oder beispielsweise Pumpen realisiert werden.

Die Reaktion wird durch Zusammenbringen des Phosgens, des inerten Lösungsmittels, welches bevorzugt erst als Lösungsmittel für das Phosgen dient, und des Phenols, welches vorzugsweise zuvor bereits in der Alkalilauge gelöst ist, gestartet. Generell wird der pH-Wert der ersten Stufe im erfindungsgemäßen Verfahren durch das Verhältnis von Alkalilauge/Phenol/Phosgen vorzugsweise so eingestellt, dass der pH-Wert im Bereich von 11,0 bis 12,0, bevorzugt 11,2 bis 11,8 liegt. Die Reaktionstemperatur wird durch Kühlung < 50°C, bevorzugt < 40°C, ganz besonders bevorzugt <35°C gehalten. Die Verweilzeit liegt im Falle des kontinuierlich geführten erfindungsgemäßen Prozesses in der ersten Stufe im Bereich von 2 Sekunden bis 300 Sekunden, bevorzugt im Bereich von 4 Sekunden bis 200 Sekunden.

In der zweiten Stufe des erfindungsgemäßen Verfahrens wird die Reaktion zum Kohlensäurediarylester komplettiert. Hier wird jetzt der Katalysator zugeführt. Direkt nach oder während der Zugabe des Katalysators wird die Reaktionsmischung im erfindungsgemäßen Verfahren gekühlt. Die Reaktionstemperatur wird durch Kühlung < 50°C, bevorzugt < 40°C, ganz besonders bevorzugt <35°C gehalten. Es kann vorteilhaft sein den Katalysator an mehreren, bevorzugt an zwei Stellen der zweiten Verfahrensstufe zuzusetzen.

Der Gehalt an Chlorameisensäurearylester im resultierenden Kohlensäurediarylester beträgt nach dem erfindungsgemäßen Verfahren < 2 ppm, bevorzugt < 0,5 ppm.

Im erfindungsgemäßen Verfahren erhält man einen Gehalt des Arylpiperidylurethans im resultierenden Kohlensäurediarylester von < 100 ppm, bevorzugt < 75 ppm, ganz besonders bevorzugt < 50 ppm.

Vorzugsweise wird in der zweiten Stufe des erfindungsgemäßen Verfahrens der pH-Wert durch Messung des pH-Werts (wird im kontinuierlichen Verfahren bevorzugt Online gemessen) und entsprechende Einstellung des pH-Wertes durch Zugabe der Alkalilauge geregelt. Die Menge zugeführter Alkalilauge wird so eingestellt, dass der pH-Wert in der zweiten Verfahrensstufe im Bereich von 7,5 bis 10,5, bevorzugt 8 bis 9 liegt.

Im erfindungsgemäßen Verfahren wird Phosgen in Bezug auf das Phenol im Verhältnis von 1,01 bis 1,15 mol-%, bevorzugt 1,05 bis 1,12 mol-% zugeführt. Das Lösungsmittel wird so zugemischt, dass der Kohlensäurediphenylester in einer 5 bis 60 %igen Lösung, bevorzugt 20 bis 45 %igen Lösung nach der Reaktion vorliegt.

Nach der Reaktion wird die organische, den Kohlensäurediarylester enthaltende, Phase üblicherweise mit einer wässrigen Flüssigkeit gewaschen und nach jedem Waschvorgang von der wässrigen Phase soweit wie möglich getrennt. Die Wäsche erfolgt bevorzugt mit entsalztem Wasser. Die Kohlensäurediarylesterlösung ist nach der Wäsche und Abtrennung der Waschflüssigkeit üblicherweise trüb. Als Waschflüssigkeit werden wässrige Flüssigkeiten, z.B. verdünnte Mineralsäuren wie HCl oder H₃PO₄, zur Abtrennung des Katalysators und zur weiteren Reinigung vollentsalztes Wasser eingesetzt. Die Konzentration von HCl oder H₃PO₄ in der Waschflüssigkeit kann beispielsweise 0,5 bis 1,0 Gew.-% betragen. Die organische Phase wird beispielhaft und vorzugsweise zweimal gewaschen.

Als Phasentrennvorrichtungen zur Abtrennung der Waschflüssigkeit von der organischen Phase können grundsätzlich bekannte Trenngefäße, Phasenseparatoren, Zentrifugen oder Coalescer oder auch Kombinationen dieser Einrichtungen verwendet werden.

Zum Erhalt des hochreinen Kohlensäurediarylester wird das Lösungsmittel abgedampft. Das Abdampfen kann in mehreren Verdampferstufen erfolgen. Beispielsweise erfolgt dies durch eine oder mehrere hintereinandergeschaltete Destillationskolonnen bei der das Lösungsmittel vom Kohlensäurediarylester getrennt wird.

Diese Reinigungsstufe bzw. Stufen können beispielsweise kontinuierlich so geführt werden, daß die Sumpftemperatur bei der Destillation > 150°C bis 310°C, bevorzugt > 160 bis 230°C liegt. Die zur Durchführung dieser Destillationen notwendigen Drücke liegen zwischen 1 und 1000 mbar, bevorzugt zwischen 5 und 100 mbar.

Die so erhaltenen Kohlensäurediester zeichnen sich aus durch eine sehr hohe Reinheit mit GC-Reinheiten (cool on column-Methode) > 99,99 %, bevorzugt 99,9925 %, ganz besonders bevorzugt > 99,995 % und einem extrem guten Umesterungsverhalten aus, so dass daraus ein Polycarbonat in excellenter Qualität hergestellt werden kann.

Die Herstellung von aromatischen Oligo-/Polycarbonaten nach dem Schmelzeumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder dem US-P 5 340 905 vorbeschrieben.

### Beispiele

### Beispiel 1

In einen gekühlten Rohrreaktor wird kontinuierlich ein Gemisch aus 117 kg/h vollentsalztes Wasser mit 48 kg/h 50 %iger NaOH und 54,9 kg/h Phenol mit einer Lösung von 98 kg/h Methylenchlorid und 31,2 kg/h Phosgen (8 mol-% Überschuss bezogen auf Phenol) zusammengebracht. Nach einer mittleren Verweilzeit von 15 Sekunden werden zu diesem Reaktionsgemisch nun in der zweiten Stufe des Verfahrens 6,5 kg/h 50 %iger NaOH zudosiert und direkt anschliessend 1,1 kg/h (0,9 %ig in Methylenchlorid) des Katalysators N-Ethylpiperidin zugefügt und das Reaktionsgemisch unmittelbar auf 30°C gekühlt. Im weiteren wird das Reaktionsgemisch durch das Durchleiten durch ein mit Verengungen versehenes Rohr ständig gemischt. Danach wird die organische von der wäßrigen Phase abgetrennt. Nach Waschen mit 0,6 %iger HCl und Wasser und abschliessender Phasentrennung wird nach dem Abdampfen des Methylenchlorids ein 99,996 %iges (GC-Methode, cool on column-Methode) Diphenylcarbonat erhalten. Der Gehalt an Phenylpiperidylurethan ist 40 ppm und der Gehalt an Chlorameisensäurephenylester liegt < 0,5 ppm.

### Vergleichsbeispiel 1

Wie Beispiel 1, nur wird die Reaktionstemperatur nach der Zugabe des Katalysators nicht gekühlt, so dass die Temperatur 55°C beträgt. Nach Waschen mit 0,6 %iger HCl und Wasser und abschliessender Phasentrennung wird nach dem Abdampfen des Methylenchlorids ein 99,97 %iges (GC-Methode, cool on column-Methode) Diphenylcarbonat erhalten. Der Gehalt an Phenylpiperidylurethan ist 300 ppm und der Gehalt an Chlorameisensäurephenylester liegt < 0,2 ppm.

### Beispiel 2

Wie Beispiel 1, nur werden 0,55 kg/h N-Ethylpiperidin (0,9 %ig in Methylenchlorid) nach 15 Sekunden zugefügt, die Reaktionslösung unmittelbar auf 30°C gekühlt und weitere 0,55 kg/h N-Ethylpiperidin (0,9 %ig in Methylenchlorid) nach 1 Minute Verweilzeit zugefügt, nachdem das Reaktionsgemisch durch das Durchleiten eines mit Verengungen versehen Rohres ständig gemischt wurde. Anschliessend wird wiederum auf 30°C gekühlt. Danach wird die organische von der wäßrigen Phase abgetrennt. Nach Waschen mit 0,6 %iger HCl und Wasser und abschliessender Phasentrennung wird nach dem Abdampfen des Methylenchlorids ein 99,998 %iges (GC-Methode, cool on column-Methode) Diphenylcarbonat erhalten. Der Gehalt an Phenylpiperidylurethan ist 20 ppm und der Gehalt an Chlorameisensäurephenylester liegt < 0,5 ppm.

## Patentansprüche

1. Zweistufiges Verfahren zur Herstellung Kohlensäurediarylestern aus Phosgen und Monophenolen mit der allgemeinen Formel 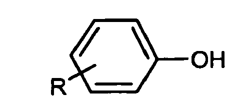 wobei R ausgewählt ist aus der Gruppe von Wasserstoff, tert-Butyl., verzweigter oder unverzweigter C₈- und/oder C₉-Alkylreste
in einem inerten Lösungsmittel in Gegenwart von Alkali und einer Stickstoffbase in der Phasengrenzfläche, **dadurch gekennzeichnet, dass** man die Temperatur in der ersten und zweiten Stufe unterhalb von 50°C hält.

2. Verfahren gemäß Anspruch 1, **dadurch gekennzeichnet, dass** man die Temperatur in der ersten und zweiten Stufe unterhalb 40°C hält.

3. Verfahren gemäß Anspruch 1, **dadurch gekennzeichnet, dass** man die Temperatur in der ersten und zweiten Stufe unterhalb 35°C hält.

4. Verfahren gemäß Anspruch 1, **dadurch gekennzeichnet, dass** als Stickstoffbase Ethylpiperidin eingesetzt wird.

5. Verfahren gemäß Anspruch 1, **dadurch gekennzeichnet, dass** das Produkt weniger als 100 ppm Arylurethane enthält.

## Claims

1. Two-stage process for producing carbonic acid diaryl esters from phosgene and monophenols with the general formula 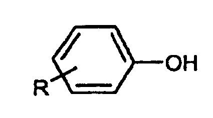 R being selected from the group of hydrogen, tert.-butyl, branched or unbranched C₈ and/or C₉ alkyl radicals
in an inert solvent in the presence of alkali and a nitrogen base in the phase interface, **characterised in that** the temperature is kept below 50 °C in the first and second stage.

2. Process according to claim 1, **characterised in that** the temperature is kept below 40 °C in the first and second stage.

3. Process according to claim 1, **characterised in that** the temperature is kept below 35 °C in the first and second stage.

4. Process according to claim 1, **characterised in that** ethylpiperidine is used as a nitrogen base.

5. Process according to claim 1, **characterised in that** the product contains less than 100 ppm aryl urethanes.

## Revendications

1. Procédé de préparation en deux phases d'esters diaryliques d'acide carbonique à partir de phosgène et de monophénols répondant à la formule générale 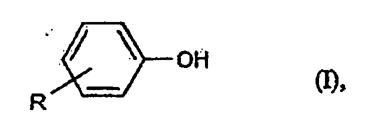 dans laquelle
R est sélectionné dans le groupe constitué par l'hydrogène, un groupe butyle tertiaire ou un radical alkyle en C₈ et/ou en C₉, ramifié ou linéaire,
dans un solvant inerte en présence d'alcalis et d'une base azotée dans l'interface, **caractérisé en ce que** l'on maintient la température dans les première et deuxième phases en deçà de 50 °C.

2. Procédé selon la revendication 1, **caractérisé en ce que** l'on maintient la température dans les première et deuxième phases en deçà de 40 °C.

3. Procédé selon la revendication 1, **caractérisé en ce que** l'on maintient la température dans les première et deuxième phases en deçà de 35 °C.

4. Procédé selon la revendication 1, **caractérisé en ce que** de l'éthylpipéridine est utilisée comme base azotée.

5. Procédé selon la revendication 1, **caractérisé en ce que** le produit contient moins de 100 ppm d'uréthanes aryliques.
